# EUROPEAN PATENT APPLICATION

(11) **EP 4 851 185 A1**
(43) Date of publication of application: **09.12.2026**
(21) Application number: 25747708.3
(22) Date of filing: 22.01.2025
(51) Int. Cl.: C07K 14/62, C07K 1/107, A61K 38/28, A61P 3/10

(54) **ULTRA-LONG-ACTING INSULIN ANALOG, PREPARATION METHOD THEREFOR, AND USE THEREOF**

(30) Priority: 04.02.2024 CN 202410159119
(71) Applicant: Lepu Jiantang Pharmaceutical (Chongqing) Co., Ltd., Chongqing 400000 (CN)
(72) Inventor: CHEN, Yong, Chongqing 400000 (CN); LUO, Hua, Chongqing 400000 (CN); LI, Xiang, Chongqing 400000 (CN); LI, Hongliang, Chongqing 400000 (CN); JIANG, Limin, Chongqing 400000 (CN); LIU, Shusheng, Chongqing 400000 (CN); PI, Shiyu, Chongqing 400000 (CN); LUO, Jihong, Chongqing 400000 (CN)
(74) Representative: Chung, Hoi Kan
(86) International application number: PCT/CN2025/074048
(87) International publication number: WO 2025/162138

(57) **Abstract**

Provided are an ultra-long-acting insulin analog, a preparation method therefor, and a use thereof. The present invention comprises modification of an amino acid sequence of natural human insulin, and by means of dual fatty acid modification as a suitable site, prolongs the acting time of an insulin analog, thereby improving patient compliance. The present invention solves the technical problem in the prior art that the drug acting time of insulin analog is unsatisfactory, uses a recombinant expression-based preparation method, and has a simple process routine and low costs.

## Description

### TECHNICAL FIELD

The present disclosure relates to the technical field of polypeptide drugs, and in particular to an ultra-long-acting insulin analog, a preparation method therefor, and a use thereof.

### BACKGROUND

Insulin is a protein hormone secreted by pancreatic β-cells in response to stimulation by endogenous or exogenous substances such as glucose, lactose, ribose, arginine, and glucagon. As an only hormone in a human body capable of lowering blood glucose, the insulin further promotes synthesis of glycogen, fat and protein. Human insulin is composed of two peptide chains, namely an A chain and a B chain. Specifically, the A chain of the human insulin contains 21 amino acids of 11 types, and the B chain contains 30 amino acids of 15 types, with a total of 51 amino acids. Sulfhydryl groups in four cysteines, namely A₇(Cys)-B₇(Cys) and A₂₀(Cys)-B₁₉(Cys), form two disulfide bonds that link the A chain and the B chain together. In addition, one disulfide bond is further formed between A₆(Cys) and A₁₁(Cys) of the A chain. To improve the acting effect and time of the insulin, extensive research has been conducted on the amino acid composition and modification methods of the insulin by researchers.

Insulin degludec, developed by Novo Nordisk, was launched in Japan in October 2012 and approved for treatment of type 1 and type 2 diabetes mellitus. As an ultra-long-acting basal insulin analog, the insulin degludec is engineered based on the human insulin by removing the threonine at a B₃₀ site and conjugating a 16-carbon dicarboxylic fatty acid to the lysine at a B₂₉ site via an L-γ-glutamic acid linker. This unique molecular structure enables the insulin degludec to exist in a stable soluble double hexamer form in preparations before injection. Nevertheless, the insulin degludec has limited in-vivo acting time and requires relatively frequent administration, which may cause certain side effects and reduce patient compliance, failing to meet clinical application requirements of the insulin. Therefore, further research on the structure and modification methods of insulin analogs is urgently needed to develop an insulin analog with longer drug acting time and a lower administration frequency.

### SUMMARY

An objective of the present disclosure is to provide an ultra-long-acting insulin analog to solve the technical problem in the prior art that the drug acting time of the insulin analog is unsatisfactory.

In order to achieve the above objective, the present disclosure employs the technical solutions as follows:
An ultra-long-acting insulin analog is provided, including an A chain: GIVEQCCTSICSLX₁QLENYCX₂X₃X₄, and a B chain: FVX₅QHLCGSHLVEALX₆LVCGERGFX₇X₈X₉X₁₀X₁₁X₁₂, where
X₁ is Y, E or D, X₂ is G, N or K, X₃ is K or absent, X₄ is A, G or absent,
X₅ is N or D, X₆ is Y, H or E, X₇ is F, H or I, X₈ is K or Y, X₉ is T, G or absent, X₁₀ is P, G, K or absent, X₁₁ is K, P or absent, and X₁₂ is T or absent.

A fatty acid is modified on an amino acid residue of X₈, X₁₀ or X₁₁.

The present technical solution further provides a preparation method for an ultra-long-acting insulin analog, including the following sequential steps:
S1: acquiring a precursor of an insulin analog A; and
S2: performing fatty acid modification on the insulin analog.

The present technical solution further provides a use of an ultra-long-acting insulin analog in preparation of drugs for treating diabetes or hypoglycemic drugs.

Further, a fatty acid is modified on an amino acid residue of X₃.

Further, the fatty acid includes a C₁₈ modifier with a structural formula shown in Formula (2), a C₂₀ modifier with a structural formula shown in Formula (3), and a C₂₂ modifier with a structural formula shown in Formula (4): 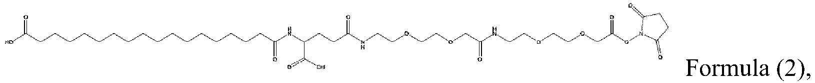 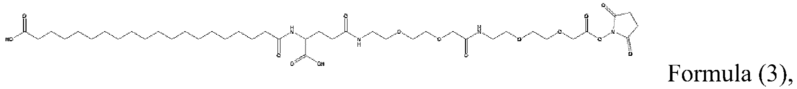 and

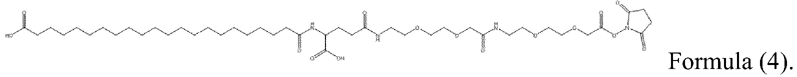

Further, X₁ is E, X₂ is N or K, X₃ is K or absent, X₄ is absent, X₅ is N, X₆ is H, X₇ is H, X₈ is Y, X₉ is T or G, X₁₀ is P or G, X₁₁ is K, X₁₂ is absent, a fatty acid is modified on an amino acid residue of X₁₁, and a fatty acid is further modified on an amino acid residue of X₂ or X₃.

Further, a sequence of the A chain is as shown in SEQ ID NO. 5 or SEQ ID NO. 7, and a sequence of the B chain is as shown in SEQ ID NO. 6 or SEQ ID NO. 8.

Further, the A chain and the B chain are connected by disulfide bonds.

Further, in the step S1, *Escherichia coli* or yeast is used for recombinant expression to obtain the precursor of the insulin analog A.

The insulin analog of the present technical solution may be obtained via expression in engineered bacteria (*Escherichia coli* and yeast). In addition, the insulin analog may further be prepared by chemical synthesis. The above preparation methods for the insulin analog are conventional means in the prior art. The synthesis or expression thereof may be carried out independently, or entrusted to biotechnology companies for synthesis and expression.

Further, a molar ratio of a modifier to the insulin analog is 3:1 to 6:1.

A fatty acid modification process includes the steps: dissolving the modifier in N-methylpyrrolidone to obtain a modifier solution, dissolving the insulin analog with sodium carbonate to obtain an insulin analog solution, and making the insulin analog solution react with the modifier solution followed by purification to obtain the fatty acid-modified insulin analog.

To sum up, the principle and the beneficial effects of the present disclosure are as follows:
The present technical solution adopts a dual-modified structure to prolong the acting time of the insulin analog and improve patient compliance. The adopted dual-modified structure is effective and prolongs the acting time. Due to higher stability of the dual-modified structure, the compound adopting the structure has highly promising for preparation of oral pharmaceutical formulations. The insulin of the present solution is prepared via a recombinant expression method with a simple process route and low costs, thereby having certain economic advantages. The present technical solution drastically prolongs the in-vivo hypoglycemic action time of the drug through rational amino acid design of the insulin and corresponding fatty acid modification.

More specifically, the technical solution newly introduces lysine (K) at a site A₂₂ of the insulin, or substitutes the amino acid residue at a site A₂₁ with lysine (K). The above sites serve as conjugation points for fatty acid modifiers to achieve fatty acid modification of the A chain. Compared with modification at a conventional glycine (G) residue at a site A₁, the operation method of the present technical solution at sites A₂₁ and A₂₂ is capable of markedly prolonging the in-vivo hypoglycemic action time of the drug. The present technical solution may further perform fatty acid modification on a site B₂₆ (requiring an amino acid residue (Y) to be substituted with lysine (K)), a site B₂₈ ((requiring an amino acid residue (P) to be substituted with lysine (K), and a site B₂₉ (being lysine (K) of the insulin, thereby achieving the fatty acid modification of the B chain, and further prolonging the in-vivo hypoglycemic action time of the drug. The present technical solution supports simultaneous dual fatty acid modification of the A chain and the B chain: one fatty acid modification site is selected from A₂₁ and A₂₂, and one fatty acid modification site is selected from B₂₆, B₂₈, and B₂₉. Such dual modification prolongs the in-vivo acting time of the drug. In addition, threonine (T) at a site B₂₇ is substituted with glycine (G), and proline (P) at the site B₂₈ is substituted with glycine (G). The above variations of the amino acid residues of the peptide chain are capable of further prolonging the in-vivo drug acting time on the basis of the fatty acid modification.

### BRIEF DESCRIPTION OF THE DRAWINGS

FIG. 1 shows mass spectrometric assay results of an Analog 2 in Embodiment 4.
FIG. 2 shows mass spectrometric assay results of an Analog 5 in Embodiment 4.
FIG. 3 shows mass spectrometric assay results of an Analog 6 in Embodiment 4.
FIG. 4 shows mass spectrometric assay results of an Analog 10 in Embodiment 4.
FIG. 5 shows mass spectrometric assay results of an Analog 11 in Embodiment 4.

### DETAILED DESCRIPTION OF EMBODIMENTS

The present disclosure is further described below in conjunction with embodiments, but the implementations of the present disclosure are not limited herein. Unless otherwise specified, the technical means used in the following embodiments and experimental examples are conventional means well known to those skilled in the art, and the materials, reagents, and the like used may be obtained from a commercial path.

The structure of an ultra-long-acting insulin analog in the present solution is shown in Formula (1):

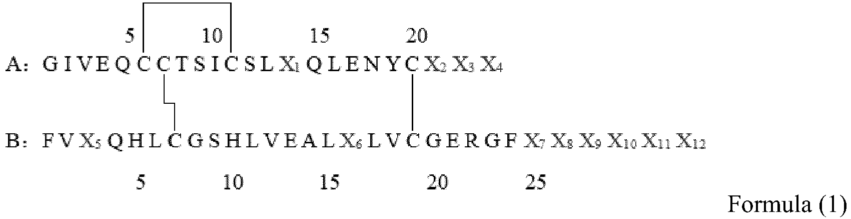

In the Formula (1), the amino acids represented by codes X₁-X₁₂ are shown in Table 1, where A denotes an A chain of insulin, and B denotes a B chain of the insulin. An amino acid sequence of the A chain is set forth in SEQ ID NO.1: GIVEQCCTSICSLnQLENYCnnn, and an amino acid sequence of the B chain is set forth in SEQ ID NO.2: FVnQHLCGSHLVEALnLVCGERGFnnnnnn. The A chain and the B chain are linked via disulfide bonds, constituting a conventional linkage method between two subunits of the insulin, and repetition is not made herein.

A sequence of human insulin is as follows: an amino acid sequence of an A chain is set forth in SEQ ID NO.3: GIVEQCCTSICSLYQLENYCN, and an amino acid sequence of a B chain is set forth in SEQ ID NO.4: FVNQHLCGSHLVEALYLVCGERGFFYTPKT.

**Table 1 Composition of ultra-long-acting insulin analog**

| [Table 1_sm_0001] | | | |
|---|---|---|---|
| Site | Code | Amino acid of natural human insulin | Amino acid of ultra-long-acting insulin analog |
| A₁₄ | X₁ | Y | Y or E or D |
| A₂₁ | X₂ | N | G or N or K |
| A₂₂ | X₃ | Absent | K or Absent |
| A₂₃ | X₄ | Absent | A or G or Absent |
| B₃ | X₅ | N | N or D |
| B₁₆ | X₆ | Y | Y or H or E |
| B₂₅ | X₇ | F | For H or I |
| B₂₆ | X₈ | Y | K or Y |
| B₂₇ | X₉ | T | T or G or Absent |
| B₂₈ | X₁₀ | P | P or G or K or Absent |
| B₂₉ | X₁₁ | K | K or P or Absent |
| B₃₀ | X₁₂ | T | T or Absent |

The dual modification sites are K residues at X₂ and X₃ on an A chain terminus as well as K residues at X₈, X₁₉, and X₁₁ on a B chain terminus. Fatty acid modifiers such as C₁₈, C₂₀, and C₂₂ may be selected, and fatty acid modification is performed at the two sites on the A chain and the B chain of the insulin. Structural formulas of the fatty acid modifiers C₁₈, C₂₀, and C₂₂ are shown in Formula (2), Formula (3), and Formula (4), respectively.

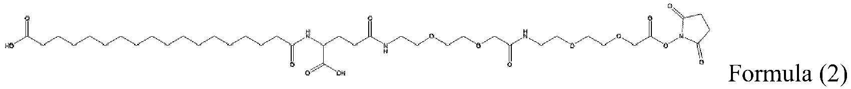

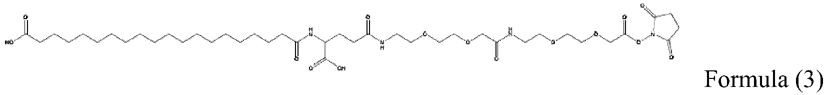

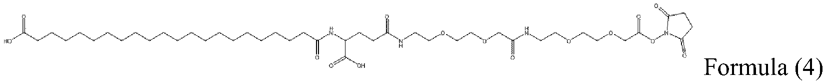

### Embodiment 1: In-vivo hypoglycemic action in normal mice

A single-dose experiment was conducted to evaluate the in-vivo hypoglycemic action time of the insulin analogs listed in Table 2 and Table 3 in normal mice. A detailed testing procedure was described as follows: SPF-grade KM mice aged 10 weeks with an average body weight of approximately 35 g were adopted, with unrestricted access to sufficient feed and drinking water. The experiment was initiated after one week of environmental acclimatization. The mice were randomly divided into a normal saline group and a drug test group with six mice per group. All the mice were injected at a dosage of 25 U/kg. The normal saline group was administered 0.1 mL per mouse as a control. Blood samples were collected from tail veins of mice at different time points after administration, and blood glucose levels were measured using Johnson & Johnson blood glucose meters. The in-vivo hypoglycemic action time of each insulin analog in the mice was determined by comparing blood glucose values between the test group and the control group. Test results are presented in Table 3.

Table 2 Base composition of insulin analogs to be tested (refer to Formula (1) for sequences of analogs, specific selection of amino acid residues is listed in this table, and "/" indicates the absence of an amino acid residue)

**[Table 1_sm_0002]**

| Site | A₁₄ | A₂₁ | A₂₂ | A₂₃ | B₃ | B₁₆ | B₂₅ | B₂₆ | B₂₇ | B₂₈ | B₂₉ | B₃₀ |
|---|---|---|---|---|---|---|---|---|---|---|---|---|
| Code | X₁ | X₂ | X₃ | X₄ | X₅ | X₆ | X₇ | X₈ | X₉ | X₁₀ | X₁₁ | X₁₂ |
| Human insulin | Y | N | / | / | N | Y | F | Y | T | P | K | T |
| Analog 1 | E | N | / | / | N | H | H | Y | T | P | K | / |
| Analog 2 | E | N | / | / | N | H | H | Y | T | P | K | / |
| Analog 3 | E | N | K | / | N | H | H | Y | T | P | K | / |
| Analog 4 | E | N | K | / | N | H | H | Y | T | P | K | / |
| Analog 5 | E | N | K | / | N | H | H | Y | T | P | K | / |
| Analog 6 | E | N | K | / | N | H | H | Y | T | P | K | / |
| Analog 7 | E | N | K | / | N | H | H | Y | T | P | K | / |
| Analog 8 | E | N | K | / | N | H | H | Y | T | P | K | / |
| Analog 9 | E | N | K | / | N | H | H | Y | G | G | K | / |
| Analog 10 | E | N | K | / | N | H | H | Y | G | G | K | / |
| Analog 11 | E | N | K | / | N | H | H | Y | G | G | K | / |
| Analog 12 | E | N | K | / | N | H | H | Y | G | G | K | / |
| Analog 13 | E | N | / | / | N | H | H | Y | T | P | K | / |
| Analog 14 | E | N | K | / | N | H | H | Y | T | P | K | / |

Table 3: Fatty acid modification profiles, administration dosages and in-vivo activity in mice of insulin analogs to be tested (amino acid compositions of the analogs are listed in Table 2, and "/" indicates no modification site selected and no fatty acid modification performed)

**Table**

| | A chain modification | | | B chain modification | | | Modification profile | In-vivo hypoglycemic action time in mice (h) |
|---|---|---|---|---|---|---|---|---|
| | Modification site | Corresponding amino acid residue | Modifier | Modification site | Corresponding amino acid residue | Modifier | | |
| Analog 1 | / | / | / | B29 | K | C₂₀ | Mono-site fatty acid modification | 40 h |
| Analog 2 | A₁ | G | C₂₀ | B29 | K | C₂₀ | Dual-site fatty acid modification | 48 h |
| Analog 3 | A22 | K | C₂₀ | / | / | / | Mono-site fatty acid modification | 30 h |
| Analog 4 | / | / | / | B29 | K | C₂₀ | Mono-site fatty acid modification | 40 h |
| Analog 5 | A₂₂ | K | C₁₈ | B₂₉ | K | C₁₈ | Dual-site fatty acid modification | 35 h |
| Analog 6 | A₂₂ | K | C₂₀ | B₂₉ | K | C₂₀ | Dual-site fatty acid modification | 56 h |
| Analog 7 | A₂₂ | K | C₂₂ | B₂₉ | K | C₂₂ | Dual-site fatty acid modification | 35 h |
| Analog 8 | / | / | / | / | / | / | / (precursor) | 2 h |
| Analog 9 | / | / | / | / | / | / | / (precursor) | 2 h |
| Analog 10 | A₂₂ | K | C₂₀ | B₂₉ | K | C₂₀ | Dual-site fatty acid modification | 80 h |
| Analog 11 | A₂₂ | K | C₂₂ | B₂₉ | K | C₂₂ | Dual-site fatty acid modification | 40 h |
| Analog 12 | / | / | / | B₂₉ | K | C₂₀ | Mono-site fatty acid modification | 48 h |
| Analog 13 | / | / | / | / | / | / | / (precursor) | 2 h |
| Analog 14 | A₂₂ | K | C₂₀ | B₂₉ | K | C₂₀ | Dual-site fatty acid modification (No Glu) | 25 h |

With reference to Table 2 and Table 3, the Analog 1 (single modification at B₂₉), Analog 2 (dual modification at A₁ and B₂₉), and Analog 13 (unmodified precursor without fatty acid modification) share consistent amino acid sequences. The A chain sequence of the analogs is set forth in SEQ ID NO.5: GIVEQCCTSICSLEQLENYCN, and the B chain sequence is set forth in SEQ ID NO.6: FVNQHLCGSHLVEALHLVCGERGFHYTPK. A site A₁ of the A chain is G, and the Analog 2 carries out fatty acid modification on an amino group of this residue.

The Analogs 3-8 and the Analog 14 have consistent amino acid sequences. The A chain sequence of the analogs is set forth in SEQ ID NO.7: GIVEQCCTSICSLEQLENYCNK, and the B chain sequence is set forth in SEQ ID NO.6: FVNQHLCGSHLVEALHLVCGERGFHYTPK. In terms of the amino acid sequence, compared with the Analog 1 (Analog 2 and Analog 13), the Analogs 3-8 and the Analog 14 are newly incorporated with lysine (K) at a site A₂₂. For the Analog 14, "No Glu" means that the fatty acid chain does not contain L-α-glutamic acid (Glu), while all other fatty acid chains contain the L-α-glutamic acid (Glu). There is no obvious difference in the synthetic processes, and the specific structure of the fatty acid for modification is shown in Formula (5).

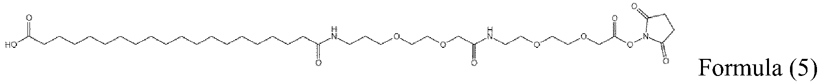

The Analogs 9-12 have consistent amino acid sequences. The A chain sequence of the analogs is set forth in SEQ ID NO.7: GIVEQCCTSICSLEQLENYCNK, and the B chain sequence is set forth in SEQ ID NO.8: FVNQHLCGSHL VEALHL VCGERGFHYGGK. In terms of the amino acid sequence, compared with the Analog 1 (Analog 2 and Analog 13), the Analogs 9-12 contain three amino acid substitutions: lysine (K) is newly introduced at a site A₂₂, threonine (T) at a site B₂₇ is substituted with glycine (G), and proline (P) at a site B₂₈ is substituted with glycine (G).

Test results of the in-vivo activity of the insulin analogs are analyzed as follows:

### (1) Insulin analogs without fatty acid modification (i.e., precursors) exhibiting short in-vivo hypoglycemic action time

Among the analogs, the Analog 8 (A chain set forth in SEQ ID NO.7, and B chain set forth in SEQ ID NO.6), the Analog 9 (A chain set forth in SEQ ID NO.7, and B chain set forth in SEQ ID NO.8) and the Analog 13 (A chain set forth in SEQ ID NO.5, and B chain set forth in SEQ ID NO.6) are all insulin analogs without fatty acid modification. When administered at the same dosage of 25 U/kg, in-vivo activity indicators of the three insulin analogs in the mice are substantially consistent (2 h). This indicates that the precursors of the insulin analogs have short in-vivo hypoglycemic action time, and fatty acid modification is required to extend the in-vivo hypoglycemic action time.

### (2) Fatty acid modification at K residues on A chain and B chain being capable of prolonging in-vivo hypoglycemic action time of drug

To prolong the duration of activity of the insulin analogs, the insulin represented by the Analog 1 was modified by C₂₀ to the K residue on the B chain of the Analog 13. Such modification plays a critical role in prolonging the duration of hypoglycemic action. The inventor further explored other fatty acid modification sites. First, fatty acid modification was performed at a site A₁ of the A chain set forth in SEQ ID NO.5, which was capable of further prolonging the in-vivo hypoglycemic action time of the drug, increasing from 40 h to 48 h compared with the Analog 1. When an amino acid residue K was added to the terminus of the A chain set forth in SEQ ID NO.5 to form the new A chain sequence set forth in SEQ ID NO.7, fatty acid modification was carried out on this K residue. A series of insulin analogs with the A chain set forth in SEQ ID NO.7 and B chain set forth in SEQ ID NO.6 (namely the Analogs 3-8 and the Analog 14) were investigated. A comparison between the Analog 2 and the Analog 6 shows that the only difference lies in C₂₀ modification at the K residue of the site A₂₂ versus C₂₀ modification at the G residue of the site A₁, and the latter prolongs the in-vivo hypoglycemic action time of the drug by 8 h relative to the former. The C₂₀ modification at the K residue of the site A₂₂ is capable of effectively enhancing the in-vivo activity of the drug in the mice and prolonging the time of the activity. Substituting the site A₂₂ of the A chain of the insulin analog with the K residue and performing fatty acid modification on this residue were attempted for the first time by the inventor, achieving a remarkable effect in prolonging the drug acting time.

### (3) Type of fatty acid modification exerting significant influence on in-vivo hypoglycemic action time of drug

The Analog 14 adopts C₂₀ modification, where the fatty acid chain does not contain L-α-glutamic acid (Glu). Compared with the Analog 6 using Glu-containing C₂₀, the in-vivo hypoglycemic action time of the Analog 14 in mice is merely 25 h, which is only about 0.45 times that of the Analog 6 (56 h).

The carbon chain length of fatty acids further has a prominent impact on the in-vivo hypoglycemic action time of the drug. Generally speaking, modifier molecules with higher molecular weights are capable of prolonging the drug circulating time in vivo, thereby prolonging the drug acting time. However, for the analogs with the A chain set forth in SEQ ID NO.7 and B chain set forth in SEQ ID NO.6 in the present technical solution, C₂₀ modification (Analog 6) greatly prolongs the in-vivo hypoglycemic action time to 56 h, while C₂₂ modification (Analog 7) and C₁₈ modification (Analog 5) only yield a duration of 35 h. A change of merely two carbon atoms brings about a 60% improvement in efficacy, and such a result was unexpected by the inventor prior to the experiment. For the analogs with the A chain set forth in SEQ ID NO.7 and B chain set forth in SEQ ID NO.8, C₂₀ modification (Analog 10) achieves in-vivo hypoglycemic action time of 80 h, whereas C₂₂ modification (Analog 11) only achieves in-vivo hypoglycemic action time of 40 h. A change of merely two carbon atoms may bring about a 50% improvement in efficacy, and such a result was unexpected by the inventor prior to the experiment. Experimental data demonstrate that screening of multiple fatty acid modifiers reveals that Glu-containing C₂₀ modification has the optimal modification performance.

### (4) Amino acid sequence of B chain exerting significant influence on in-vivo hypoglycemic action time of drug modified by fatty acid

The inventor adjusted the B chain to obtain the analogs with A chain set forth in SEQ ID NO.7 and B chain set forth in SEQ ID NO.8, and attempted fatty acid modification thereon. Specifically, on the basis of SEQ ID NO.6 of the B chain, the two amino acid residues TP are substituted with GG. Under the identical C₂₀ modification, the Analog 6 (with amino acid residues TP) has an in-vivo hypoglycemic action time of 56 h, while the Analog 10 (with amino acid residues GG) has in-vivo hypoglycemic action time of 80 h. Under the identical C₂₂ modification, the Analog 7 (with amino acid residues TP) has an in-vivo hypoglycemic action time of 35 h, while the Analog 11 (with amino acid residues GG) has in-vivo hypoglycemic action time of 40 h. A comparison between the Analog 10 and the Analog 6 reveals that the substitution of two amino acid residues drastically prolong the in-vivo hypoglycemic action time by 24 h at an improvement rate of 43%, which was also unexpected by the inventor prior to the experiment. Comparing the Analog 11 and the Analog 7, for C₂₂ modification, the amino acid sequence of the A chain set forth in SEQ ID NO.7 combined with the B chain set forth in SEQ ID NO.8 presents superior performance. Although the substitution of the two amino acid residues do not produce a more dramatic improvement than that observed with C₂₀ modification, the in-vivo hypoglycemic action time still increases by 5 h at an improvement rate of 14%. For single fatty acid modification targeting the K residue at the site B₂₉, comparison between the Analog 12 (amino acid residues GG) and the Analog 4 (amino acid residues TP) shows that the Analog 12 achieves longer drug acting time, with an extension of 8 h and an improvement rate of 20%. Therefore, the analogs with the A chain set forth in SEQ ID NO.7 and B chain set forth in SEQ ID NO.6 and the analogs with the A chain set forth in SEQ ID NO.7 and B chain set forth in SEQ ID NO.8 only differ by two amino acids at the terminal of the B chain, yet the two types of analogs exhibit markedly different effects after fatty acid modification. In particular, for dual C₂₀ fatty acid modification, the B chain set forth in SEQ ID NO.8 achieves an unexpected technical effect.

To sum up, compared with the Analog 1 (Analog 2 and Analog 13), the Analogs 9-12 contain three amino acid substitutions: lysine (K) is newly introduced at the site A₂₂, threonine (T) at the site B₂₇ is substituted with glycine (G), and proline (P) at the site B₂₈ is substituted with glycine (G). Among the analogs, after amino acid substitutions at the sites B₂₇ and B₂₈, the analogs with single modification at the site B₂₉ exhibit the significantly prolonged time of activity, and the analogs with dual modification at the sites A₂₂ and B₂₉ exhibit the significantly prolonged time of activity. Lysine (K) at the site A₂₂ may provide a new modification site, and the analogs with dual modification at the sites A₂₂ and B₂₉ show longer time of activity when compared to those with dual modification at the sites A₁ and B₂₉.

In addition to the aforementioned sequences (Analogs 9-12), based on the human insulin, an N residue at the terminal X₂ (A₂₁) of the A chain may be substituted with the residue K, a Y residue at the terminal X₈ (B₂₆) of the B chain may be substituted with the residue K, and a P residue at the terminal X₁₀ (B₂₈) of the B chain may be substituted with the residue K, thereby introducing dual modification sites (K residues serve as conjugation sites for fatty acid modifiers), and generating novel insulin sequences. Dual fatty acid modification performed on these novel insulin sequences may further achieve effects comparable to those of the Analog 10.

### Embodiment 2: Recombinant expression of insulin analogs in Escherichia coli

According to an amino acid sequence of an insulin analog A (with reference to the amino acid sequences of the insulin analogs containing the A chain and the B chain that are set forth in Table 1 and Table 2), a cDNA sequence of the analog was designed by adopting codons preferred by the *Escherichia coli*, and full-length gene synthesis was completed by Sangon Biotech (Shanghai) Co., Ltd. The synthesized cDNA fragment was inserted into a pET-26b(+) plasmid, followed by double digestion with Nde I and Hind III and fragment recovery. The recombinant fusion expression pET-26b(+) plasmid was subjected to identical double digestion followed by large fragment recovery. The gene fragment of the insulin analog A was ligated to the pET-26b(+) plasmid under the action of a T4 ligase followed by transformation into a TOP10 strain. Recombinant plasmids containing the inserted insulin analog A gene were obtained through plate screening and designated pET-26b-A. Subsequently, the recombinant plasmid was transformed into an expression host strain BL21(DE3) using a CaCl₂ method to obtain a recombinant expression strain. Fusion protein of the insulin analog A was produced via induction with 1 mmol/L IPTG. The fusion protein was subjected to inclusion body recovery, refolding and ion exchange chromatography, followed by enzymatic digestion. The product was further purified via reversed-phase chromatography and lyophilized to yield an insulin analog A precursor. A specific preparation process of the insulin analog A precursor (i.e., insulin analog without fatty acid modification) was described as follows: after recombinant expression in the *Escherichia coli*, bacterial cells were collected by high-speed centrifugation, resuspended in water, homogenized at 60 MPa, and centrifuged to obtain inclusion bodies of the insulin analog A. The inclusion bodies of the insulin analog were solubilized at a mass-to-volume ratio of 1:10 in lysis buffer (8 mol/L urea, 20 mmol/L β-mercaptoethanol, and 20 mmol/L Tris, with pH of 10.0). The mixture was stirred until precipitates were fully dissolved, then centrifuged to prepare an insulin analog A fusion protein solution. The solution was diluted with pre-cooled water at 2-8°C at a volume ratio of 1:10, and statically refolded at 2-8°C for 40 h. Dilute hydrochloric acid was added to adjust the pH to 5.0-6.0, followed by static precipitation for 2 h, and centrifugation to recover the insulin analog A fusion protein. Dissolution was performed in 50 mmol/L Tris (pH 9.0), and enzymatic digestion was performed at 25°C for 8 h. A C8 reversed-phase chromatographic column was adopted, with a mobile phase A composed of 20% acetonitrile and 0.1% TFA and a mobile phase B composed of 60% acetonitrile. Gradient elution was performed to obtain a precursor of the insulin analog A.

### Embodiment 3: Recombinant expression of insulin analogs in Pichia pastoris

According to an amino acid sequence of an insulin analog A, a cDNA sequence of the analog was designed by adopting codons preferred by the *Pichia pastoris*, and full-length gene synthesis was completed by Sangon Biotech (Shanghai) Co., Ltd. The synthesized cDNA fragment was inserted into a pPICZαA plasmid, followed by double digestion with Not I and Xho I and fragment recovery. The recombinant fusion expression pPICZαA plasmid was subjected to identical double digestion followed by large fragment recovery. The gene fragment of the insulin analog A was ligated to the pPICZαA plasmid under the action of a T4 ligase followed by transformation into a TOP10 strain. Recombinant plasmids containing the inserted insulin analog A gene were obtained through plate screening and designated pPICZαA-A. Subsequently, the recombinant plasmid was transformed into an expression host strain X-33 using an electrotransformation method to obtain a recombinant expression strain. Fusion protein of the insulin analog A was produced via induction with a methanol-glycerol mixture. The fusion protein was subjected to ion exchange chromatography, followed by enzymatic digestion. The product was further purified via reversed-phase chromatography to yield a recombinant insulin analog A followed by lyophilization into dry powder. After recombinant expression in the *Pichia pastoris*, bacterial cell supernatant was collected via high-speed centrifugation. Cation exchange chromatography was adopted with a mobile phase A composed of 20 mmol/L sodium acetate (pH 3.0) and a mobile phase B composed of 20 mmol/L sodium acetate and 0.5 M NaCl (pH 3.0). Fusion protein of THE insulin analog A was obtained after ion-exchange purification. Enzymatic digestion was performed at 25°C with pH of 8.0 for 8 h. A C8 reversed-phase chromatographic column was adopted, with gradient elution carried out by a mobile phase A composed of 20% acetonitrile and 0.1% TFA, and a mobile phase B composed of 60% acetonitrile to obtain a precursor of the insulin analog A. This embodiment serves as a general method for preparing the precursor of the insulin analog A. Subsequent modification was then performed on the obtained precursor.

### Embodiment 4: Modification of insulin analog precursor with modifiers

Mono-modified and dual-modified samples are mainly obtained by controlling a modification ratio, which is a conventional technical means in the prior art. Under the pH of 9.5 to 11.5, the modifier preferentially modifies sites A₂₂ and B₂₉, and two types of samples may be separated by reversed-phase chromatography in this case. When the modification ratio of the insulin analog precursor to the modifier is 1:4, A₂₂ and B₂₉ dual-modified samples are acquired and recovered via reversed-phase chromatography. To obtain the A₂₂ mono-modified analog (Analog 3), the modification molar ratio of the insulin analog precursor to the modifier was controlled within 1:1.5 to 1:2.5. To obtain the B₂₉ mono-modified analogs (Analog 4 and Analog 12), the modification molar ratio of the insulin analog precursor to the modifier was controlled within 1:1.5 to 1:2.5. Target samples were then separated by reversed-phase chromatography.

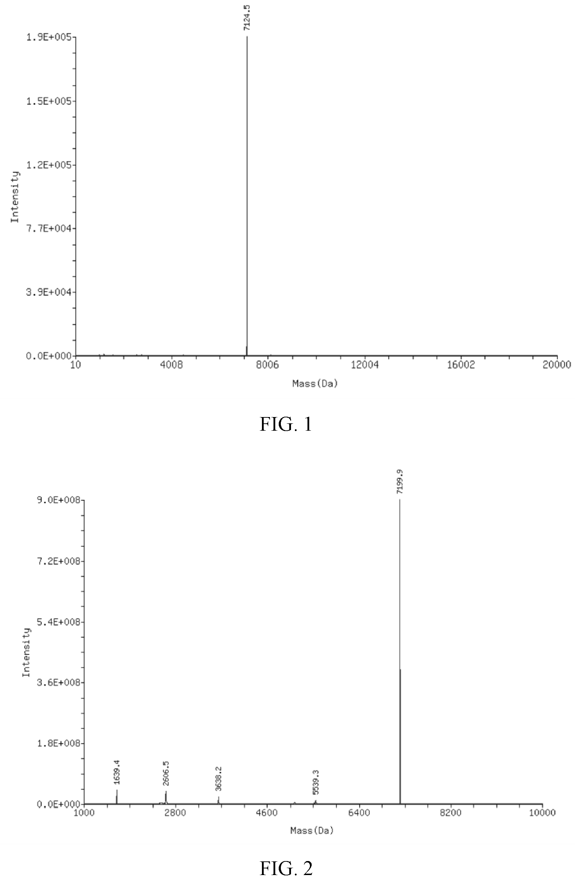

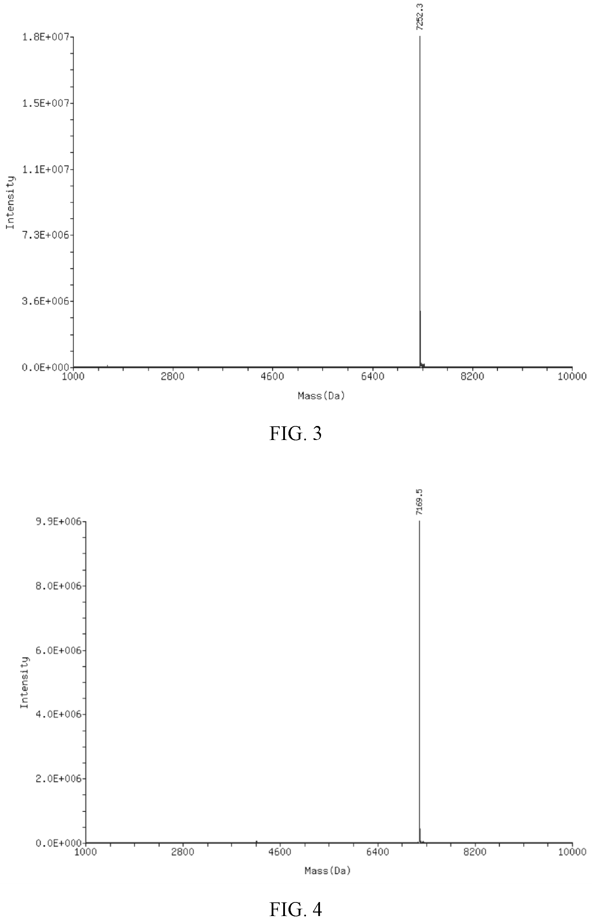

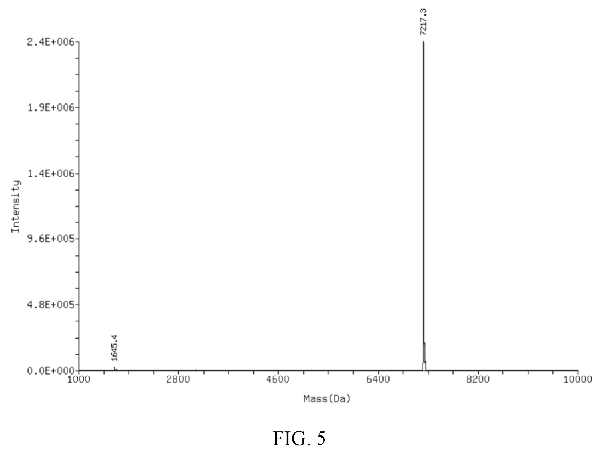

The modifier (C₁₈, C₂₀ or C₂₂) was dissolved in N-methylpyrrolidone to a concentration of 100 mg/mL. A certain amount of the insulin analog precursor was weighed and dissolved in a 0.1 mol/L sodium carbonate solution to 10 mg/mL, and the pH was adjusted to 10.5-11.5. Under stirring at room temperature, the modifier was added at a molar ratio of the modifier to the insulin analog precursor of 4:1 to 6:1 for a reaction for 0.5 h. Then, the pH was adjusted to 7.5 with acetic acid. After completion of the reaction, purification was performed by reversed-phase chromatography to yield insulin analogs, and the obtained insulin analogs were preserved by lyophilization. Molecular weight information was acquired via LC-MS analysis. In addition, a synthesis method for the modifier (C₁₈, C₂₀ or C₂₂) may be referenced to the prior patent CN116655770A (Fatty acid-modified thrombopoietin mimetic peptide homotetramer, preparation method therefor, and use thereof). The molecular weight information of the Analogs 1-14 synthesized by the present solution is shown in Table 4. Mass spectrometry detection results of the Analogs 2, 5, 6, 10, and 11 are presented in FIG. 1 to FIG. 5.

**Table 4 Molecular weight information of Analogs 1-14**

| Table | | | | | | |
|---|---|---|---|---|---|---|
| Name | A chain | | B chain | | Modification profile | Molecular weight |
| | Modification profile | Sequence | Modification profile | Sequence | | |
| Analog 1 | / | SEQ ID NO.5 | B₂₉, K, C₂₀ | SEQ ID NO.6 | Mono-site fatty acid modification | 6381.0 |
| Analog 2 | A₁, G, C₂₀ | SEQ ID NO.5 | B₂₉, K, C₂₀ | SEQ ID NO.6 | Dual-site fatty acid modification | 7124.5 |
| Analog 3 | A₂₂, K, C₂₀ | SEQ ID NO.7 | / | SEQ ID NO.6 | Mono-site fatty acid modification | 6511.8 |
| Analog 4 | / | SEQ ID NO.7 | B₂₉, K, C₂₀ | SEQ ID NO.6 | Mono-site fatty acid modification | 6511.8 |
| Analog 5 | A₂₂, K, C₁₈ | SEQ ID NO.7 | B₂₉, K, C₁₈ | SEQ ID NO.6 | Dual-site fatty acid modification | 7199.9 |
| Analog 6 | A₂₂, K, C₂₀ | SEQ ID NO.7 | B₂₉, K, C₂₀ | SEQ ID NO.6 | Dual-site fatty acid modification | 7252.3 |
| Analog 7 | A₂₂, K, C₂₂ | SEQ ID NO.7 | B₂₉, K, C₂₂ | SEQ ID NO.6 | Dual-site fatty acid modification | 7308.3 |
| Analog 8 | / | SEQ ID NO.7 | / | SEQ ID NO.6 | / (precursor) | 5767.4 |
| Analog 9 | / | SEQ ID NO.7 | / | SEQ ID NO.8 | / (precursor) | 5682.9 |
| Analog 10 | A₂₂, K, C₂₀ | SEQ ID NO.7 | B₂₉, K, C₂₀ | SEQ ID NO.8 | Dual-site fatty acid modification | 7169.5 |
| Analog 11 | A₂₂, K, C₂₂ | SEQ ID NO.7 | B₂₉, K, C₂₂ | SEQ ID NO.8 | Dual-site fatty acid modification | 7217.3 |
| Analog 12 | / | SEQ ID NO.7 | B₂₉, K, C₂₀ | SEQ ID NO.8 | Mono-site fatty acid modification | 6427.8 |
| Analog 13 | / | SEQ ID NO.5 | / | SEQ ID NO.6 | / (precursor) | 5636.7 |
| Analog 14 | A₂₂, K, C₂₀ | SEQ ID NO.7 | B₂₉, K, C₂₀ | SEQ ID NO.6 | Dual-site fatty acid modification | 6958.3 |

### Embodiment 5: Hypoglycemic test of type 1 diabetic mouse models

### (1) Modeling of mice

Type 1 diabetic mouse models were constructed by intraperitoneal injection of streptozotocin (STZ). Mice with blood glucose levels above 16.8 mmol/L were regarded as successfully modeled.

### (2) Drug administration to mice

The modeled mice were divided into three groups: a model group, an Analog 1 group, and an Analog 6 group. Normal mice were set as a blank control group. The normal group and the model group were administered PBS, with 6 administrations in total and 0.2 mL of PBS per dose. An administration frequency of the Analog 1 was once per 36 hours, and an administration frequency of the Analog 6 was once per 36 hours. The dosage of administration for the two analogs was 25 U/kg. After 6 consecutive administrations, blood glucose levels of the mice in each group were measured at 0 h, 12 h, 24 h, 36 h, 48 h, 72 h, 78 h, 84 h, and 96 h after administration. Measurement lasted until the blood glucose levels of the drug-treated groups returned to a baseline level before administration. Test data are listed in Table 5. When the Analog 1 was adopted, the blood glucose level was recovered to the baseline level before administration at 72 h. In contrast, when the Analog 6 was adopted, the blood glucose level remained below the baseline level before administration at 96 h after administration, indicating that the Analog 6 possesses longer acting time and a better effect.

**Table 5: Blood glucose level measurement results (mmol/L)**

| [0082] [Table 1_sm_0003] | | | | | | | | | |
|---|---|---|---|---|---|---|---|---|---|
| Group | 0 h | 12 h | 24 h | 36 h | 48 h | 72 h | 78 h | 84 h | 96 h |
| Model group | 22.9 | 23.7 | 24.8 | 25.1 | 23.1 | 24.3 | 22.6 | 22.6 | 22.6 |
| Blank group | 8.4 | 8.7 | 8.3 | 9.7 | 8.5 | 8.3 | 8.1 | 8.1 | 8.1 |
| Analog 1 | 12.0 | 2.2 | 1.9 | 3.2 | 17.8 | 25.2 | - | - | - |
| Analog 6 | 6.9 | 9.7 | 3.2 | 6.2 | 4.3 | 3.5 | 6.7 | 9.1 | 10.5 |

### Embodiment 6: Hypoglycemic test of type 1 diabetic rat models

Rats were acclimatized for 1 week with free access to food and water. After all indicators passed inspection, the rats were fasted for 12 h with free access to water, followed by tail vein injection of 1% streptozotocin (STZ) at a dose of 55 mg/kg. During injection, STZ was dissolved in citrate buffer (pH of 4.5) to at a concentration of 1%. The corresponding STZ was administered according to a fasting body weight of each rat, and all injections were completed within 30 minutes. Model establishment criteria: Fasting blood glucose levels (6-hour fasting with free water intake) was measured 3 days after administration once daily for 3 consecutive days, and the rats with the fasting blood glucose levels being ≥ 16.8 mmol/L were regarded as successfully established type 1 diabetic models.

According to the fasting blood glucose levels and body weight, the rats were stratified and randomly divided into a vehicle control group, a positive control group and multiple test drug groups, with 9-10 rats per group. Subcutaneous injection was adopted for administration at an injection volume of 1 mL/kg. The day of the first administration was recorded as Day 0, and the administration cycle lasted for 6 weeks.

Diabetic rats received multiple subcutaneous injections of peptide Analog 10 at 1.8 mg/kg and Analog 6 at 2 mg/kg. LC-MS was adopted to determine plasma concentrations of the drug at 3, 12, 24, 36, 48, 60, 72, 96, 120, 144, 168, 192 and 216 h after the last administration. The measured results of the parent drug in plasma of each test animal at different time points are shown in Table 6. All test data were expressed as Mean ± Standard Deviation (Mean ± SD). GraphPad Prism 8.3 analysis software was applied for data analysis. Detailed descriptions of parameters listed in Table 6 are as follows:

t_{1/2} (elimination half-life): The time required for a plasma concentration of the drug to decrease to half of an initial value, and this parameter is a critical indicator to evaluate an elimination rate of the drug in vivo.

Tₘₐₓ (time to maximum concentration): The time taken for the drug to reach a maximum concentration (Cₘₐₓ) in blood after administration, and this parameter may be used to assess an absorption rate of the drug entering blood circulation. Cₘₐₓ (maximum plasma concentration): A maximum concentration value of the drug in blood, and this parameter represents a highest exposure level of the drug in vivo, and is vital for assessing the safety and efficacy of the drug.

AUC₀₋₂₁₆ₕ (Area Under the Curve from 0 to 216 h): The total area under a plasma drug concentration-time curve ranging from administration to 216 h after administration, and this value is used to estimate the total drug exposure within this period.

AUC_{0-∞} (Area Under the Curve from time zero to infinity): Similar to AUC₀₋₂₁₆ₕ, this parameter calculates the overall drug exposure throughout an entire process from administration until theoretical complete elimination of the drug, and this parameter reflects the overall exposure of the drug more comprehensively.

MRT₀₋₂₁₆ₕ (Mean Residence Time from 0 to 216 h): The mean residence time of drug molecules in vivo from administration to 216 h after administration, and this parameter is an indicator to evaluate how long the drug remains in vivo.

MRT_{0-∞} (Mean Residence Time from time zero to infinity): Similar to MRT₀₋₂₁₆ₕ, but this parameter covers the whole time range of drug presence until complete clearance from the body, offering a more complete overview of drug residence time in vivo.

**Table 6 Summary and comparison of pharmacokinetic parameters in diabetic rats after multiple administrations**

| [Table 1_sm_0004] | | | |
|---|---|---|---|
| Parameter | Unit | Analog 10 (1.8 mg/kg) | Analog 6 (2 mg/kg) |
| t_{1/2} | h | 40±14.9 | 32±12.5 |
| Tₘₐₓ | h | 12±15.2 | 18±13.9 |
| Cₘₐₓ | ng/mL | 15200±6690 | 17400±9050 |
| AUC₀₋₂₁₆ₕ | h·ng/mL | 1070000±588000 | 1300000±979000 |
| AUC_{0-∞} | h·ng/mL | 1110000±622000 | 1350000±1050000 |
| MRT₀₋₂₁₆ₕ | h | 53.7±14.2 | 53.2±14.2 |
| MRT_{0-∞} | h | 60.1±20.4 | 57.7±19.1 |

### Embodiment 7: Affinity test of insulin analogs

Determination was performed using a surface plasmon resonance (SPR) method. The affinity of insulin derivatives provided by the present disclosure to a human insulin receptor (IR) and an insulin-like growth factor 1 receptor (IGF-1R) was determined. In this test, the SPR method was adopted to test the affinity of samples. Two analytical models, namely "Steady state affinity" and "1:1 binding", are available in analysis software. The specific model was selected according to the dissociation profile of each sample. In the test, insulin derivatives were immobilized on chips: The receptors were diluted to 25 µg/mL with acetate (pH of 4.0). A volume of 180 µL was loaded, and a channel 2 of a flow cell 1 on the chip was selected for an amine coupling immobilization test. Subsequently, kinetic parameters were set as follows: Receptors (IR/ IGF-1R) were diluted to 250 nM in 1×PBS buffer (pH of 6), then serially diluted to concentrations of 125 nM, 62.5 nM, 31.25 nM, 15.625 nM, 7.8125 nM and 3.90625 nM. A flow rate was set to 20 µL/min, association time was 60 s, and dissociation time was 90 s. A reaction temperature was maintained at a default value of 25°C before sample loading and detection. Association and dissociation were analyzed using the analytical model of "Steady state affinity" and/or "1:1 binding" to calculate (equilibrium dissociation constant) KD values. In this embodiment, affinity tests and studies were performed on a commercial insulin, an Analog 1, an Analog 6 and an Analog 10. Detailed test results are presented in Table 7 and Table 8. Test results demonstrate that the affinity constant between the insulin receptor and the insulin is 336 nM, while the affinity constant of tested insulin analogs ranges from 52.3 nM to 213 nM. The affinity constant between the insulin-like growth factor 1 receptor and the insulin is 1.43 µM, while the affinity constant of the tested insulin analogs ranges from 19.3 nM to 39.1 nM.

**Table 7: Affinity constant between insulin receptor and tested insulin analogs**

| [0098] [Table 1_sm_0005] | | | | |
|---|---|---|---|---|
| Polypeptide | Ka | Kd | KA | KD |
| Insulin | / | / | / | 3.36E-07 |
| Analog 1 | 6.45E+03 | 1.38E-03 | 4.67E+06 | 2.13E-07 |
| Analog 6 | 1.96E+04 | 2.63E-03 | 7.45E+06 | 1.34E-07 |
| Analog 10 | 1.58E+04 | 8.26E-04 | 1.91E+07 | 5.23E-08 |

**Table 8: Affinity constant between insulin-like growth factor 1 receptor and tested insulin analogs**

| [Table 1_sm_0006] | | | | |
|---|---|---|---|---|
| Polypeptide | Ka | Kd | KA | KD |
| Insulin | / | / | / | 1.43E-06 |
| Analog 1 | 1.37E+05 | 4.98E-03 | 2.75E+07 | 3.63E-08 |
| Analog 6 | 5.79E+04 | 1.12E-03 | 5.17E+07 | 1.93E-08 |
| Analog 10 | 3.35E+04 | 1.31E-03 | 2.56E+07 | 3.91E-08 |

### Embodiment 8: In-vitro activity test of insulin analogs

Stable cell lines and luciferase detection kits were adopted to measure the cellular bioactivity in vitro. Preliminary test results demonstrated that binding of insulin or analogs of the insulin to a human insulin receptor (INSR) expressed on the cell line surface rapidly triggers receptor activation, followed by activation of other downstream signaling pathway components. This further initiated transcription and translation of the luciferase reporter gene stably integrated into the chromosome. After cell lysis, a luciferase detection reagent was added to generate robust chemiluminescence signals. The detected signal intensity exhibited a tight dose-dependent correlation with the bioactivity of the tested insulin samples. Based on this cellular detection platform, in-vitro pharmacodynamic dose-response curves of insulin may be plotted to compare the relative bioactivity (potency) between samples to be tested and a reference standard.

HEK293/Luc/INSR cells were cultured in a medium (DMEM+10% FBS) and maintained in a humidified incubator at 37°C with 5% CO₂. Cells were passaged via trypsin digestion. When cell confluence reached 80%-90% and cell viability exceeded 90%, the cells were ready for plating. The cell density was adjusted to 5×10⁵ cells/mL using a medium (DMEM+10% FBS), and 20 µL of cell suspension (10000 cells per well) was dispensed into each well of a 384-well plate. A PBS solution (50 µL per well) was added to surrounding peripheral wells. The cell plate was then incubated overnight in an incubator at 37°C with 5% CO₂. The cell plate was taken out from the incubator, and 20 µL of a sample working solution was added to each well, with 3 replicate wells set for each concentration. Preparation of working solution: An aliquoted reference standard stock solution was removed from a refrigerator at -20°C and equilibrated to room temperature. In a 96-well U-bottom plate, the reference standard stock was diluted to 44 µM with the medium (DMEM+10% FBS), followed by serial 2-fold dilutions to prepare a total of 10 concentration gradients. The cell plate was returned to the incubator for a further incubation for 6 h. Raw data in Excel format were exported from PHERAstar. GraphPad Prism was launched, concentrations of the reference standard were converted to logarithmic values, which were plotted on an X-axis against relative light units (RLU) on a Y-axis. Four-parameter logistic fitting was performed to calculate a median effective concentration (EC₅₀) value of the reference standard. Test results of EC₅₀ values of human insulin, Analog 6, and Analog 1 are summarized in Table 7. Specifically, the EC₅₀ values of the human insulin, the Analog 6, and the Analog 1 are 3.047×10⁻⁹, 1.261×10⁻⁶, and 1.099×10⁻⁶, respectively. The human insulin herein refers to a commercial batch (Novolin R, JVGT586).

**Table 9 Test results of EC₅₀ values of human Insulin, Analog 6, and Analog 1**

| Table | | | |
|---|---|---|---|
| log(agonist) vs. response - Variable slope (four parameters) | | | |
| Best-fit values | Human insulin | Analog 6 | Analog 1 |
| Bottom | 2384 | 1523 | 871.2 |
| Top | 29224 | 40764 | 46169 |
| LogEC50 | -8.516 | -5.899 | -5.959 |
| HillSlope | 2.415 | 1.581 | 1.460 |
| EC50 | 3.047e-009 | 1.261e-006 | 1.099e-006 |
| Span | 26840 | 39241 | 45297 |

What are described are only embodiments of the present disclosure, and common knowledge such as specific technical solutions and/or characteristics that are well known in the solution are not described too much here. It should be noted that for those skilled in the art, they may also make several transformations and improvements on the premise of not deviating from the technical solutions of the present disclosure, and these transformations and improvements shall be regarded to fall within the protection scope of the present disclosure and will not affect the implementation effect of the present disclosure and the practicability of patent. The protection scope claimed by the present disclosure shall be subject to the contents of the claims, and the particular implementations and other descriptions in the description may serve to interpret the contents of the claims.

## Claims

1. An ultra-long-acting insulin analog, **characterized by** comprising an A chain: GIVEQCCTSICSLX₁QLENYCX₂X₃X₄, and a B chain: FVX₅QHLCGSHLVEALX₆LVCGERGFX₇X₈X₉X₁₀X₁₁X₁₂, wherein
X₁ is Y, E or D, X₂ is G, N or K, X₃ is K or absent, and X₄ is A, G or absent;
X₅ is N or D, X₆ is Y, H or E, X₇ is F, H or I, X₈ is K or Y, X₉ is T, G or absent, X₁₀ is P, G, K or absent, X₁₁ is K, P or absent, and X₁₂ is T or absent; and
a fatty acid is modified on an amino acid residue of X₈, X₁₀ or X₁₁.

2. The ultra-long-acting insulin analog according to claim 1, **characterized in that** a fatty acid is further modified on an amino acid residue of X₂ or X₃.

3. The ultra-long-acting insulin analog according to claim 2, **characterized in that** the fatty acid comprises a C₁₈ modifier with a structural formula shown in Formula (2), a C₂₀ modifier with a structural formula shown in Formula (3), and a C₂₂ modifier with a structural formula shown in Formula (4): 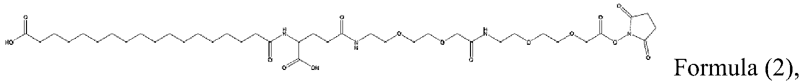 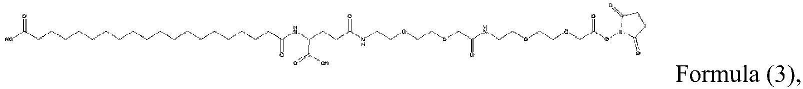 and

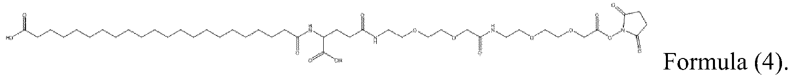

4. The ultra-long-acting insulin analog according to claim 3, **characterized in that** X₁ is E, X₂ is N or K, X₃ is K or absent, X₄ is absent, X₅ is N, X₆ is H, X₇ is H, X₈ is Y, X₉ is T or G, X₁₀ is P or G, X₁₁ is K, X₁₂ is absent, a fatty acid is modified on an amino acid residue of X₁₁, and a fatty acid is further modified on an amino acid residue of X₂ or X₃.

5. The ultra-long-acting insulin analog according to claim 4, **characterized in that** a sequence of the A chain is as shown in SEQ ID NO. 5 or SEQ ID NO. 7, and a sequence of the B chain is as shown in SEQ ID NO. 6 or SEQ ID NO. 8.

6. The ultra-long-acting insulin analog according to claim 5, **characterized in that** the A chain and the B chain are connected by disulfide bonds.

7. A preparation method for the ultra-long-acting insulin analog according to any one of claims 1-6, **characterized by** comprising the following steps:
S1: acquiring a precursor of an insulin analog A; and
S2: performing fatty acid modification on the insulin analog.

8. The preparation method for the ultra-long-acting insulin analog according to claim 7, **characterized in that** in the step S1, *Escherichia coli* or yeast is used for recombinant expression to obtain the precursor of the insulin analog A.

9. The preparation method for the ultra-long-acting insulin analog according to claim 7, **characterized in that** in the step S2, a molar ratio of a modifier to the insulin analog is 3:1 to 6:1; and a fatty acid modification process comprises the steps: dissolving the modifier in N-methylpyrrolidone to obtain a modifier solution, dissolving the insulin analog with sodium carbonate to obtain an insulin analog solution, and making the insulin analog solution react with the modifier solution followed by purification to obtain the fatty acid-modified insulin analog.

10. A use of the ultra-long-acting insulin analog according to any one of claims 1-6 in preparation of drugs for treating diabetes or hypoglycemic drugs.
